# EUROPEAN PATENT SPECIFICATION

(11) **EP 3 727 476 B1**
(45) Date of publication and mention of the grant of the patent: **22.03.2023**
(21) Application number: 18891447.7
(22) Date of filing: 21.12.2018
(51) Int. Cl.: G06K 7/10, A61L 2/10, F21V 23/04, F21V 23/00, G06T 7/73, A61L 2/08, A61L 2/24, G07G 1/00

(54) **DATA COLLECTION DEVICE WITH ANTI-MICROBIAL ILLUMINATION**
DATENERFASSUNGSVORRICHTUNG MIT ANTIMIKROBIELLER BELEUCHTUNG
DISPOSITIF DE COLLECTE DE DONNÉES DOTÉ D'UN ÉCLAIRAGE ANTIMICROBIEN

(30) Priority: 22.12.2017 US 201715852252
(43) Date of publication of application: 28.10.2020
(73) Proprietor: Datalogic USA, Inc., Eugene, OR 97402-9150 (US)
(72) Inventor: HARDIN, Wesley, W., Eugene, OR 97402 (US); MONTE, Matt, Eugene, OR 97402 (US); THOMPSON, Ryan, Eugene, OR 97402 (US)
(74) Representative: Porta & Consulenti Associati S.p.A.
(86) International application number: PCT/US2018/067135
(87) International publication number: WO 2019/126666

(56) References cited:
- US-A1- 2008 283 769
- US-A1- 2008 297 767
- US-A1- 2011 290 889
- US-A1- 2015 071 819
- US-A1- 2016 271 659
- US-A1- 2017 296 686

## Description

### FIELD OF THE INVENTION

The present invention relates to barcode readers, and more specifically, to barcode readers having a cleaning or disinfecting function for disinfecting surfaces of workspaces.

### BACKGROUND OF THE INVENTION

Barcode readers are used in a variety of venues. The purpose of a barcode reader can vary greatly from venue to venue as well as within the venue. In retail environments, a venue may have multiple barcode readers at a single station or point-of-sale for performing various functions. For example, a venue may have a first barcode reader for scanning barcodes on products, a second barcode scanner for reading checks and other forms of payment, and a third barcode reader for identifying objects in a basket. Each barcode reader provides different functions that use different configurations, such as illumination, field-of-view, and reading capabilities. It should be understood that barcode readers are capable of reading machine-readable indicia other than barcodes, including two-dimensional codes, such as QR codes.

Moving a single barcode reader from one orientation to another is time and resource intensive as a user often has to manually modify each of the settings affected by the new orientation and related functionality. Time and personnel are valuable resources to meet demand. As a result, venues install multiple barcode readers with a purpose of each barcode reader stationary in its orientation (or be dedicated to being operated in a hand-held mode), thus removing the need to ever change settings and functionality. Even though the use of multiple barcode readers is less costly in time and personnel resources, the cost of obtaining and maintaining extra barcode readers is a burden on the venue.

Barcode readers are often used within environments in which harmful germs and bacteria exist. For example, barcode readers are used in grocery stores in which foods and chemicals are placed on scanning or processing areas (e.g., workspace surfaces) at the barcode readers. As another example, barcode readers are used in hospitals and other environments in which harmful germs and bacteria exist. As an example, in a grocery store, food products, such as chicken grease, may contact a work surface, such as a conveyer belt, barcode scanner surface, or other surface. As such, reducing harmful germs and bacteria in food handling applications, hospitals, and other environments is highly desirable. Currently, cleaning chemicals and expensive anti-microbial materials are used to control/clean the scanning areas or workspace surfaces, including user interfaces, at the barcode readers. The workspace surfaces could be cleaner, safer, and more efficient while reducing the use of expensive and potentially harmful chemicals. Many of the chemicals that are used to disinfect surfaces are harmful and/or degrade materials (e.g. polycarbonate/ABS) commonly found in the workspace.

US 2008/0297767 A1 discloses a hand-held reader of electro-optically reading indicia, such as bar code symbols or other marking, wherein the reader includes a housing which accommodates UV light sources and safety means for reducing exposure to the UV light beam generated by the UV light sources. The safety means comprise an orientation sensor for determining an orientation of the housing in which the UV light sources are activated.

US 2008/0283769 A1 discloses a medical imaging apparatus comprising a frame whereon is mounted at least one medical imaging data processing unit connected to imaging sensor forming means. The frame comprises at least one integrated chamber for disinfecting the imaging sensor forming means. The integrated chamber comprises means for generating a disinfecting radiation and adapted to implement a sanitizing cycle thereof.

### BRIEF SUMMARY OF THE INVENTION

In a first aspect thereof, the present invention relates to a code reader as recited in claim 1.

The barcode or code reader of the invention is configured to automatically adjust at least one setting in order to perform a functionality corresponding to a detected orientation of the barcode reader. In response to a sensor sensing a new orientation, the barcode reader automatically selects a corresponding function. The automatic selection of a function according to orientation may be performed in real time so that a process performed by the barcode reader and corresponding workstation may have limited, or no, interruption of operation. The barcode reader is connected to a stand of a workstation or barcode reader system of a point-of-sale (POS) so that orientation is determined based on a configuration of a housing of the barcode reader. The housing may be detachably connected to the stand so that detaching of the barcode reader indicates a hands-free mode of operation. In an alternative embodiment, rather than being responsive to a change in orientation, the barcode reader may determine orientation in response to a request or activation of a reading function and adjust functionality and/or settings at that time based on the determined orientation. Orientation may alternatively be determined based on trained image position sensing in which analysis of the background of the imager's current field-of-view determines function of the scanner based on a comparison of pre-established images set during a "training" mode in which specific behavior is established in relationship to an image background (e.g., top surface of a scanner system).

One orientation corresponds with a cleaning function. The cleaning function includes emitting an anti-microbial illumination that is used to clean or disinfect surface(s) onto which the barcode scanner illuminates with the anti-microbial illumination. The anti-microbial illumination is an illumination with a wavelength that causes microbes (e.g., bacteria) to be deactivated or killed. The cleaning function may correspond with more than one orientation, and may include emitting (i) a first power level of the anti-microbial illumination to coincide with emitting an illumination for reading a barcode in a first orientation and (ii) a second power level of the anti-microbial illumination in a second orientation for higher intensity cleaning, where the first power level may be lower than the second power level. Additionally and/or alternatively, the anti-microbial illumination may be turned on automatically, semi-automatically, and/or manually on a periodic (e.g., once or more times per day) or aperiodic (e.g., in response to an event, such as a protection screen being pulled over a surface area on which the barcode scanner is configured to illuminate with the anti-microbial illumination). In addition to the anti-microbial illumination being produced by an illuminator (e.g., LED illumination between approximately 380 and about 470nm wavelengths that produce blue light) of the barcode scanner, illumination devices (e.g., LEDs) may be disposed on poles or light fixtures arranged to illuminate the surface(s) with the anti-microbial illumination at which the barcode scanner operates.

In a second aspect thereof, the present invention relates to a method for disinfecting a target area at a code reader as recited in claim 9.

One embodiment of a method out of the present invention for disinfecting a surface at a code reader may include sensing an environmental factor at the code reader. In response to determining that the environmental factor is at a first value, the code reader may be configured to read machine-readable indicia. In response to determining that environmental factor is at a second value, an anti-microbial illumination may be caused to be emitted toward a target area of the scanner.

### BRIEF DESCRIPTION OF THE DRAWINGS

Illustrative embodiments of the present invention are described in detail below with reference to the attached drawing figures, which are incorporated by reference herein and wherein:
**FIG. 1** is an illustration of an illustrative barcode reader system configured to read machine-readable indicia utilizing a code reader out of the invention inside a housing having a set of orientations;
**FIG. 2** is an illustration of an illustrative housing of a code reader out of the invention in four different orientations;
**FIG. 3** is an illustration of an illustrative code reader out of the invention in a fifth orientation, which is a handheld scanning orientation;
**FIG. 4** is an illustration of an illustrative barcode reader system having a code reader out of the invention configured to perform trained image position sensing to support orientation identification;
**FIG. 5** is an illustration of an illustrative barcode reader system having a code reader out of the invention inclusive of an electromechanical sensor used to determine orientation;
**FIG. 6** is an illustration of an illustrative machine-readable indicia scanner for use in scanning machine-readable indicia, such as barcodes, QR codes, or other machine-readable indicia along with physical dimensions of products or packaging by a code reader out of the invention;
**FIG. 7** is a block diagram of illustrative components of a code reader system that selects a function for a code reader according to the invention based on an orientation thereof;
**FIG. 8** is a block diagram of illustrative modules performed by a code reader system configured to automatically select functions based on orientation and perform barcode reading using a selected function by a code reader according to the invention;
**FIG. 9** is a flow diagram of an illustrative method of automatically selecting a function of a code reader according to the invention based on an orientation thereof;
**FIG. 10** is a flow diagram of an illustrative method of automatically selecting a function of a code reader according to the invention based on an orientation thereof;
**FIGS. 11A** and **11B** are illustrations of illustrative code readers according to the invention in orientations corresponding with a cleaning function;
**FIGS. 12A** and **12B** are illustrations of an illustrative top-down reader according to the invention inclusive of a light source configured to emit an anti-microbial illumination for cleaning;
**FIGS. 13A-13C** are illustrations of an illustrative code reader according to the invention inclusive of a reflective roller shade for use in a cleaning function;
**FIGS. 14A** and **14B** are illustrations of an illustrative scanner inclusive of an image sensor and a light source of a code reader according to the invention configured to be used in cleaning functions and scanning functions;
**FIG. 15** is an illustration of an illustrative handheld code reader out of the invention having a substantially transparent housing;
**FIG. 16** is an illustration of an illustrative code reader out of the invention inclusive of a conveyor belt with overhead scanner and having a cleaning function;
**FIG. 17** is an illustration of an illustrative code reader station out of the invention, with an overhead anti-microbial illumination source for disinfecting a workspace of the station; and
**FIG. 18** is a flow diagram of an illustrative method of automatically selecting a function of a code reader according to the invention based on an orientation thereof.

### DETAILED DESCRIPTION OF THE INVENTION

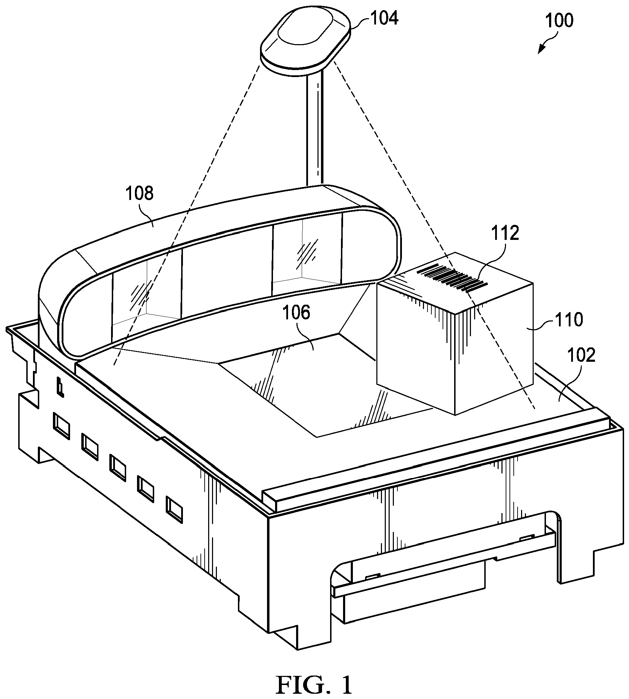

With regard to **FIG. 1****,** an illustration of a barcode reader system **100** including a base **102** and code reader **104** configured to read machine-readable indicia is shown. The code reader **104** may be configured to be positioned in multiple orientations. The barcode reader system **100** may further include a first barcode scanner **106,** and a second barcode scanner **108** to enable the barcode reader system **100** to read machine-readable indicia from three different angles to provide efficiency in reading a machine-readable indicia on an object, such as a consumer package. The barcode reader system **100** may be configured to identify item **110** by reading a machine-readable indicia **112** in a target area within a field-of-view of the barcode reader system **100.** The machine-readable indicia **112** may be representative of a code (e.g., UPC code) associated with the item **110** that enables the barcode reader system **100** to identify the items for checkout at a store or other purpose. "Barcode" may refer to a "barcode," "code," or any other machine-readable indicia as known by one of skill in the art.

In one embodiment, the barcode reader system **100** may be configured to constantly scan the target area, such as, but not limited to, a scanning station. In response to identifying the existence of an item **110** in the target area, the first and second barcode scanners **106** and **108** and the code reader **104** may scan or image the machine-readable indicia **112** (e.g., barcode, QR code, or any other machine-readable code or markings) captured on the item **110.** The barcode reader system **100** may have a variety of alternative configurations, as understood in the art.

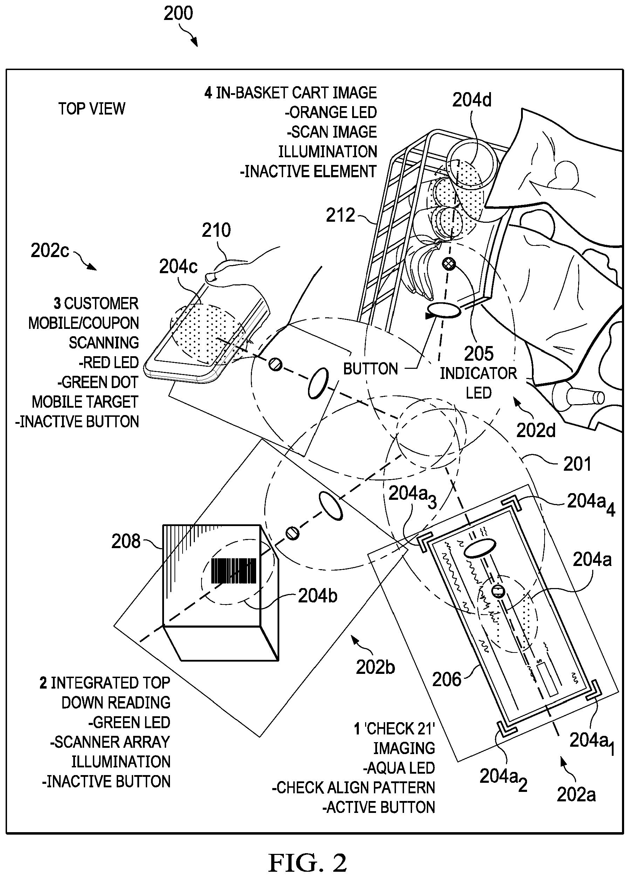

With regard to **FIG. 2****,** an illustration of a barcode reader **200** having a housing **201** of a code reader is shown in four different mutually different orientations **202a - 202d** (collectively **202**). In one embodiment, the housing **201** includes a light source (not shown) that may illuminate a target area. In one embodiment, the housing **201** may include a target indicator light (not shown) that may provide a positioning spot or other indicator (e.g., rectangle corners **204a₁**-**204a₄** (collectively **204a**) **204a - 204d** (collectively **204**) to indicate to a user where to place an item for reading. If different target indictor lights are used to support different functions of the barcode reader **104,** then the barcode reader **104** may include a driver system (e.g., processor, optical source driver, etc.) to automatically select and control the appropriate light source(s) to turn on and off the light source(s) based on the selected function. In addition to a light source, a function indicator light **205** may be used to notify a user of the selected function. In one embodiment, the indicator light **205** may produce different colors (e.g., red, green, blue, violet), and each function may cause the function indictor light **205** to produce a different color. Alternatively, the function indicator light **205** may be composed of multiple lighting elements associated with different physical positions on the housing **201** to indicate different functions.

To operate the barcode scanner **200,** the scanner **200** may be set to an automatic scan mode to continuously scan, scan in response to an event (e.g., identification of motion), periodically scan (e.g., scan every 0.1 seconds), or manually scan in response to a user pressing a button **207** to initiate a scan. In response to pushing the button **207,** the scanner **200** may automatically determine a function based on orientation (e.g., angular, inclination, motion, background image content) of the scanner **200.** Alternatively, the scanner **200** may be configured for a particular function based on orientation prior to a user pushing the button **207** (e.g., in response to a change in orientation, the scanner changes function, thereby being configured to a particular function prior to activation of a scan request by a user or automatic scanning, as previously described).

The barcode reader **200** may include different functions corresponding to the respective four orientations **202** of the housing **201.** The functions may be automatically selected by adjusting at least one setting of the barcode reader **200.** The settings may include, but are not limited to, depth of field, region of interest, type of data captured, type and format of data transmitted (e.g., fully decoded barcode data in a case of a customer facing scanner function versus time-synced frame contents in the case of a top-down reader, and non-synced images in a case of an in-basket scanner and check imager, such as a Check 21 imager). Transition from one function to another may be achieved dynamically in real-time or near-real-time so that transmission from the code reader is seemingly, or actually, instantaneous to avoid usage disruption or delay.

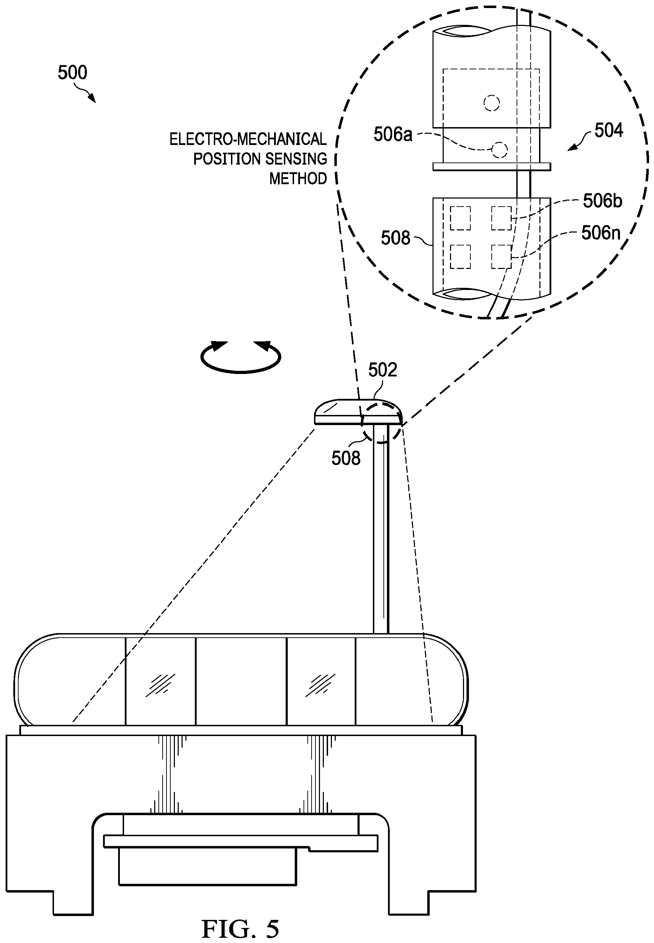

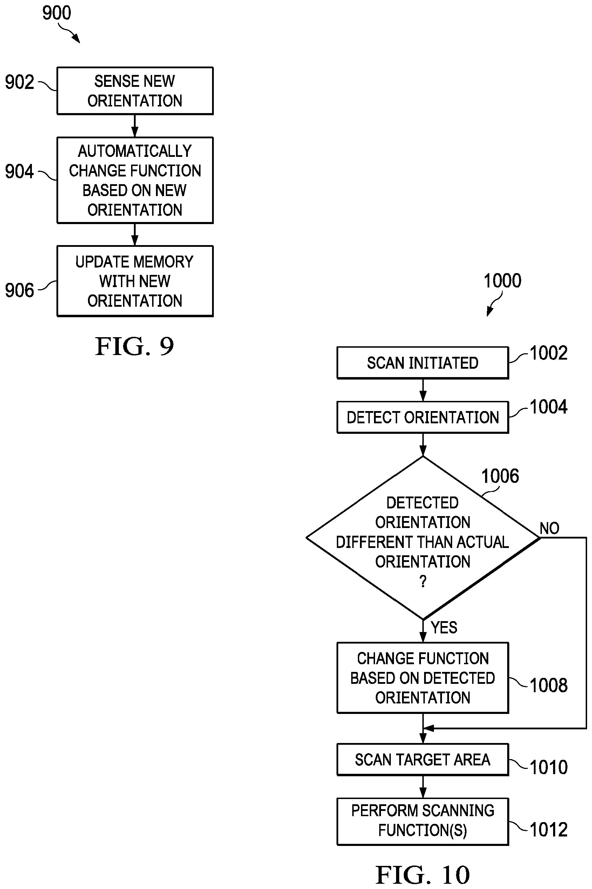

The barcode reader **200** may automatically change functions in response to a change in orientation of the housing **201.** In one embodiment, a change in orientation is sensed by at least one electromechanical sensor, such as shown in **FIGS. 5** and **9****,** and communicated to the barcode reader **200** so that the barcode reader **200** may automatically select a function corresponding to the orientation of the housing **201.** In one embodiment, a scan may initiated, such as shown in **FIG. 10****,** and the orientation of the housing **201** may be determined initially by an image captured of a background portion of the target area. For example, determining an orientation of the housing **201** based on the image of the target area may include identifying known features (e.g., scanning window or a base of a barcode scanner system) in a background portion of the image of the target area that correspond with a function corresponding with the orientation of the housing **201.** The barcode reader **200** is shown in four orientations **202** as an example, and an alternative number of orientations corresponding with different functions may be supported.

A first orientation **202a** of the housing **201** may be a check imaging orientation. A function of the barcode reader **200** in the check imaging orientation may be an imaging function. The barcode reader **200** may capture an image of a check **206** that a user has placed in the target area. In an embodiment, the barcode reader **200,** in response to the barcode reader **200** entering into a check imaging function, one or more illumination devices may be turned on to define a region in which a check (or other item) may be placed. The illumination devices may define a rectangle in which the check is to be placed, for example, thereby supporting specific functionality of processing checks, which generally has a higher degree of image processing than barcode scanning. The imaging function may include auto-sizing and meeting pixel requirements for check processing applications, such as, for example, Check 21 processing. The imaging function may include parallax corrections as well as other imaging corrections for reading and verifying checks known to one of skill in the art.

In one embodiment, the orientations **202** may be determined by angular or other (e.g., inclination) orientation of the housing **201.** In another embodiment, the orientations may be determined by identifying a background portion (e.g., outline on a base, sticker, text, or other fixed feature in the target area). Orientation may be determined by a variety of alternative methods, as further described herein.

A second orientation **202b** of the housing **201** may be a barcode scanner orientation. A function of the barcode reader **200** in the barcode scanner orientation **202b** may be to constantly, periodically, or aperiodically scan the target area. In response to identifying an item **208** in the target area, the code reader may scan or image a machine-readable indicia disposed on the item **208.** The barcode reader **200** may communicate the image to a POS system optionally to be processed with images captured by additional scanners below and to a side of the item **208** (see **FIG. 1****,** for example). The barcode reader **200** may also be configured to identify a code associated with the item **208** by reading the machine-readable indicia and communicating the code associated with the item **208** to an external processing unit (e.g., POS system). Orientation of the housing **201** may be determined by at least those methods as described hereinabove with reference to the check imaging orientation as well as identifying a machine-readable indicia in the target area.

A third orientation **202c** of the housing **201** may be a mobile device reader orientation for reading a machine-readable indicia displayed on a screen of a mobile device **210.** A function of the barcode reader **200** in the mobile device reader orientation **202c** may be image processing that has different settings than those used to read barcodes on items. The barcode reader **200** may read or capture an image on the mobile device **210.** The barcode reader **200** may then transmit the image to an external processing unit. The barcode reader **200** may provide a positioning spot **204c** to indicate to a user where to place the mobile device **210** to optimize reading the screen of the mobile device **210.** The screen of the mobile device **210** may display an image of a number of illustrative items related to point-of-sale transactions, such as, but not limited to, coupons, payment information, venue membership information, and other items known to those of skill in the art for use in purchasing or paying for items. Orientation may be determined by at least those methods as described hereinabove with reference to the check imaging orientation as well as identifying a mobile device or an illuminated screen in the target area.

A fourth orientation **202d** of the housing **201** may be an in-basket orientation for scanning contents of a basket **212.** A function of the barcode reader **200** in the in-basket orientation **202d** may be image processing as well as changing a focal distance or field-of-view settings or parameters of the barcode reader **200.** The barcode reader **200** may capture and process images of the basket **212** to determine a variety of parameters, such as, but not limited to, how many items remain in the basket **212,** if any items are hidden underneath other items in the basket **212,** how many of a same item are in the basket **212** to speed up check-out time, and other parameters known to those with skill in the art.

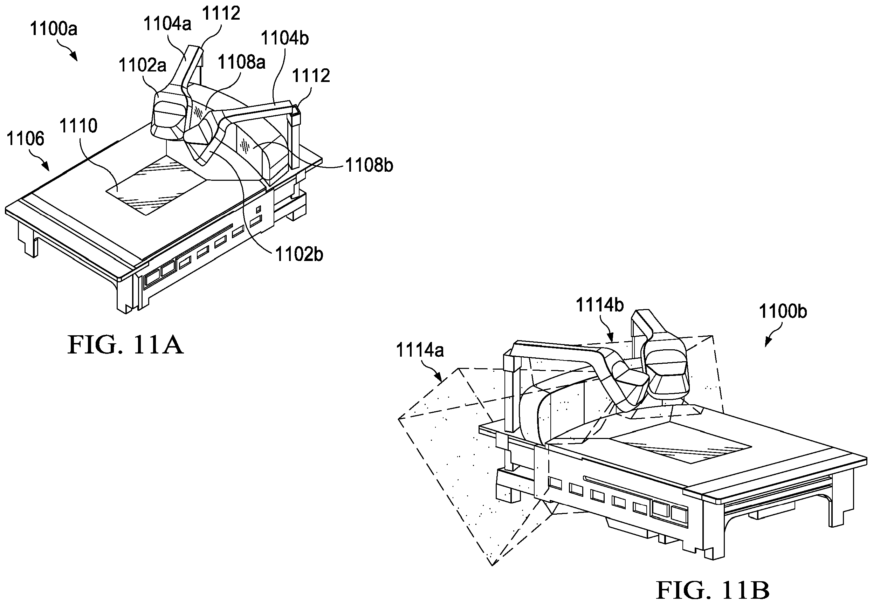

A fifth orientation (not shown) of the housing **201** may be a cleaning orientation for cleaning the barcode reader **200.** A function of the barcode reader **200** in the cleaning orientation may be a cleaning function including cleaning working surfaces and scanners of the barcode reader **200.** The housing **201** may be configured to emit an anti-microbial illumination during the cleaning function and may be described in further detail hereinbelow with regard to **FIGS. 11A-18****.** The cleaning function may also correspond with the first orientation **202a,** the second orientation **202b,** and the third orientation **202c** in addition to, or independent of, the fifth orientation.

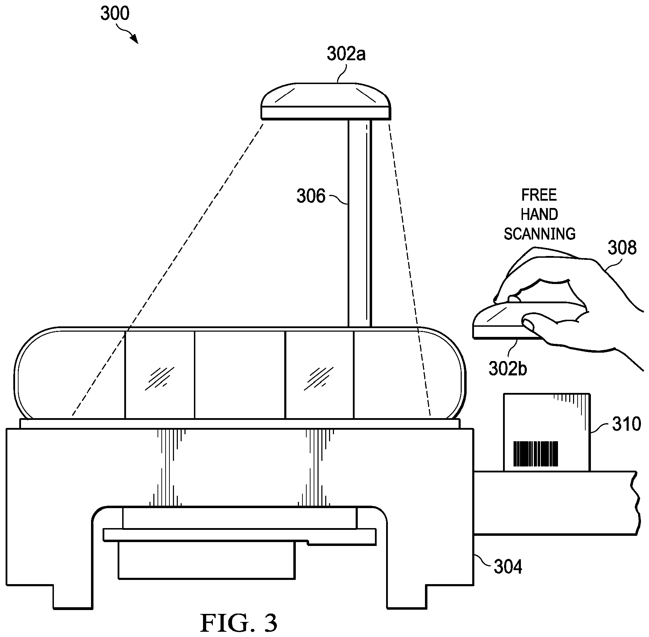

With regard to **FIG. 3****,** an illustration of a barcode reader system **300** having a barcode reader **302a** and **302b** (collectively **302**) in a handheld scanning orientation **303** is shown. The barcode reader system **300** may include a base **304** that may operate as a portion of a POS, and to which a support stand **306** may be connected. The barcode reader **302** may be detached, by a user **308,** from the support stand **306** for use in a handheld reading function mode. The barcode reader **302b** in the handheld reading function mode may allow the user **308** to scan or image an item **310** that is not within a field-of-view of the code reader **302a** in an attached orientation when positioned on the support stand **306.**

In one embodiment, a function of the code reader **302** in the handheld scanning orientation **303** may be image processing and data storage. The code reader **302** may be configured to scan a target area for an item inclusive of a machine-readable indicia. In one embodiment, the barcode reader **302b** in a handheld reading function mode may transmit an image of the target area back to the base **304,** for example, a point-of-sale station, through a wireless communication system, such as, but not limited to, Wi-Fi^{®}, Bluetooth^{®}, NFC^{®}, wired, or other communication methods known to those of skill in the art.

In another embodiment, the barcode reader **302b** in the handheld mode may store any gathered information from the item **310** in local memory. In an embodiment, the locally stored data may be transmitted to the base **304** when the code reader **302b** is re-attached to the support stand **306.** The stored data may include product information, venue information read from a barcode (e.g., UPC barcode), item count, item sizes, images, OCR data, as well as other data relevant to consumer goods.

The handheld scanning orientation **303** may be determined by any of the processes described hereinabove with reference to **FIG. 2****,** as well as other methods one of skill in the art will appreciate with regard to handheld devices. For example, in one embodiment, orientation may be determined by a gravimeter disposed within a housing of the barcode reader **302.** Alternatively and/or additionally, accelerometer(s) to sense motion, inclinometer to sense inclination, or other motion or orientation sensor may be utilized. In one embodiment, the barcode reader **302** may sense detachment from the base **304.** In one embodiment, a power feed may be disconnected at detachment and the barcode reader **302** may sense a lack of power feed and/or beginning to use power local to the barcode reader **302,** such as, but not limited to, a battery, a capacitor, a set of capacitors, a super capacitor, or any other power supply method known to those of skill in the art. A motion or other sensor may alternatively be used to sense that the barcode reader **302** is in a handheld scanning orientation **303.** In an embodiment, a determination that the barcode reader **302** is in the orientation **303** may be performed in response to being detached from the support stand **306,** in response to being moved, or commanded to perform a scan, as further described herein.

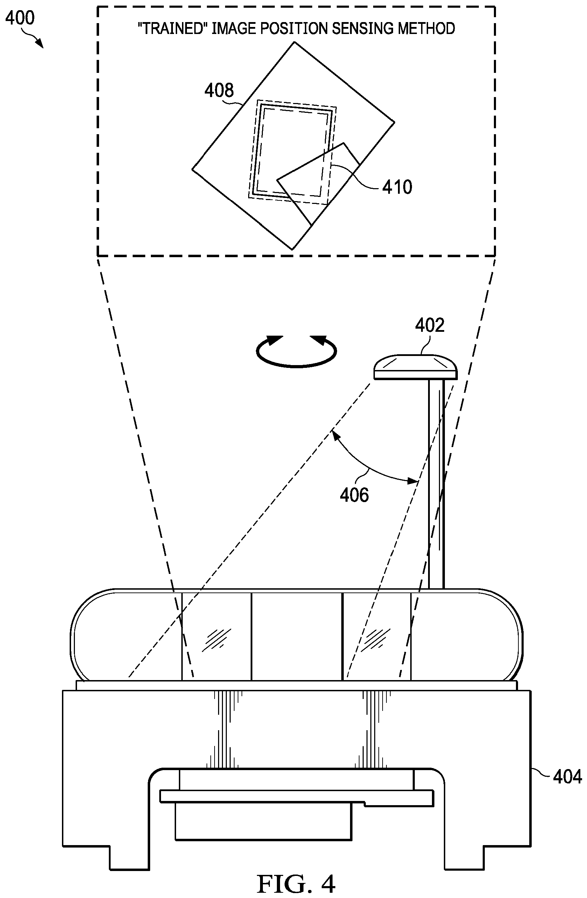

With regard to **FIG. 4****,** an illustration of a barcode reader system **400** having a barcode reader **402** configured to perform scanning when in a "trained" image position to support orientation identification is shown. The barcode reader system **400** may include the barcode reader **402** and a base **404,** as previously described with respect to **FIG. 3****.** The barcode reader **402** may be configured to have a field-of-view **406** in which the barcode reader **402** may scan for items. The base **404** may include at least one template **408** that the barcode reader **402** has been trained to identify as corresponding with an orientation. The barcode reader **402** may include a guide template **410** that may assist in aligning with the template **408** as well as orientation detection.

In one embodiment, one or more orientations of a housing of the barcode reader **402** may include a respective template that the barcode reader **402** may be trained to identify. The different trained templates may include different configurations (e.g., markings, fixed features, such as scan window edges, stickers, or otherwise) so that the barcode reader **402** may identify a corresponding orientation based on the configuration of the trained template **408.**

With regard to **FIG. 5****,** an illustration of a barcode reader system **500** having a barcode reader **502** inclusive of an electromechanical sensor **504** formed of electrodes **506a** - **506n** (collectively **506**), positioned on a support stand **508,** and used to determine orientation is shown. Rather than using electrodes, alternative sensing devices, such as optical sensors or otherwise, may be utilized to enable the barcode reader **502** to determine orientation of the reader **502.** The electrodes **506** may provide voltage signal(s) that the barcode reader **502** may measure to determine angular orientation of the barcode reader **502.** In an embodiment, as the barcode reader **502** is rotated, different signals may be read from a configuration of the electrodes **506.** For example, the electrodes **506** may be configured such that when the different electrodes **506** are contacted by a set of complimentary electrodes in electrical communication with the barcode reader **502,** a respective binary signal is communicated or sensed by the barcode reader **502.**

In one embodiment, the electromechanical sensors **508** may be include a predetermined number of sensors configured so that varying angular orientations of the housing cause different electrodes or sensors to align so that the orientation signal may represent a configuration of the electrodes **506** and a corresponding orientation. The stand of the base **504** and the connecting mechanism may house a wire that electrically couples the barcode reader **502** to the base **504.** In one embodiment, the wire may transmit a power source from the base **504** to the barcode reader **502.** In one embodiment, the wire may also be configured to support data communications between the barcode reader **502** and the base **504.**

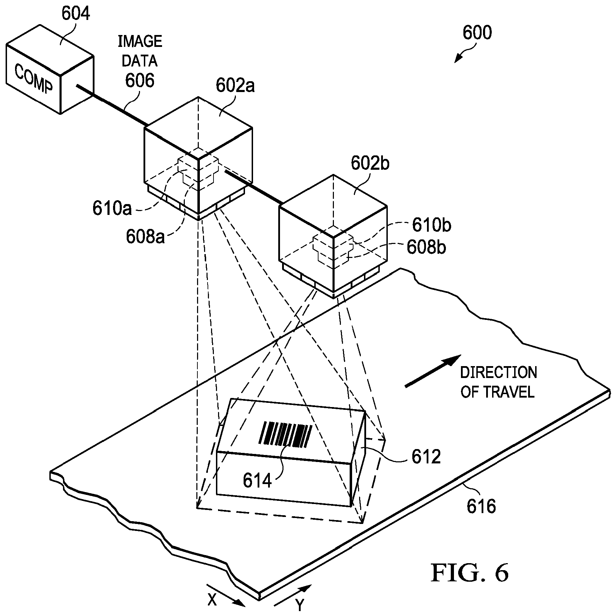

With regard to **FIG. 6****,** an illustration of a machine-readable indicia scanner system 600 for use in scanning machine-readable indicia, such as barcodes, QR codes, or other machine-readable indicia along with physical dimensions of products or packaging is shown. The scanner system **600** may additionally and/or alternatively be configured to operate as a product inspection system or logistics processing system. The scanner system **600** may include cameras **602a** and **602b** (collectively **602**) configured to capture images of an object **612,** and generate image data **606** and/or data (e.g., codes) representative of the machine-readable indicia. The cameras **602** may include optics **608a** and **608b** (collectively **608**), which may include lens(es), window, optical filter(s), and so on, and image sensors **610a** and **610b** (collectively **610**) may be used for imaging a scene. In an embodiment, the image data **606** captured by the cameras **602** may be communicated to a computer system **604** for processing (e.g., reading a code from a machine-readable indicia) thereby. Alternatively, the cameras **602** may be configured with processing units (not shown) to process image data **606** and generate data derived therefrom (e.g., text representative of machine-readable indicia). In an embodiment, the cameras **602** and computer system **604** may be formed as single units. In one embodiment, the cameras **602** are configured (e.g., spatially aligned) so that the image data **606** from camera **602a** and camera **602b** may combine to produce a three dimensional image, as understood in the art. Although the scanner system **600** shows two cameras **602,** a single camera or more than two cameras may be utilized to capture images from similar or different angles.

In an embodiment, the cameras **602** may identify markings, such as words, stickers, or features on a conveyer belt **616** that cause the cameras **602** to automatically enter a certain function or establish certain parameter(s).

As shown, an object **612** on which a machine-readable indicia **614** is positioned on the conveyer belt **616** that operates to move the object **612** along a direction of travel of the conveyer belt **616.** When the cameras **602** image the object **612,** the optics **608** and image sensors **610** may have some level of blur in the image, thereby being problematic for conventional image processing, as previously described. Depending on height of the object **612,** speed of the conveyer belt **616,** resolution of a machine-readable indicia **614** associated with, in this case attached to, the object **612,** illumination of the indicia **614,** optical noise, and so on may also contribute to difficulty in reading or decoding the indicia **614** by conventional image processing techniques. As a result, a processing unit of the computer system **608** may be configured to automatically utilize an algorithm that generates a virtual scanline in response to recognition of certain system parameters and machine-readable indicia type (acting as different "orientations" and corresponding "functions") to be able to more accurately determine or decode codewords of the machine-readable indicia.

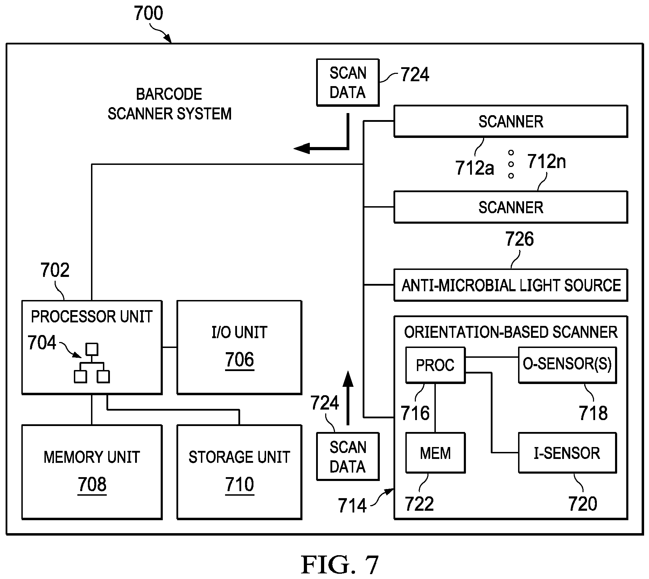

With regard to **FIG. 7****,** a block diagram of components of a code reader system **700** that selects a function for a code reader based on an orientation thereof is shown. The code reader system **700** may include a processing unit **702,** an input/output (I/O) unit **706** for communicating data, such as image data, a memory unit **708,** a storage unit **710,** scanners **712a** - **712n** (collectively **712**), and an orientation-based scanner **714.**

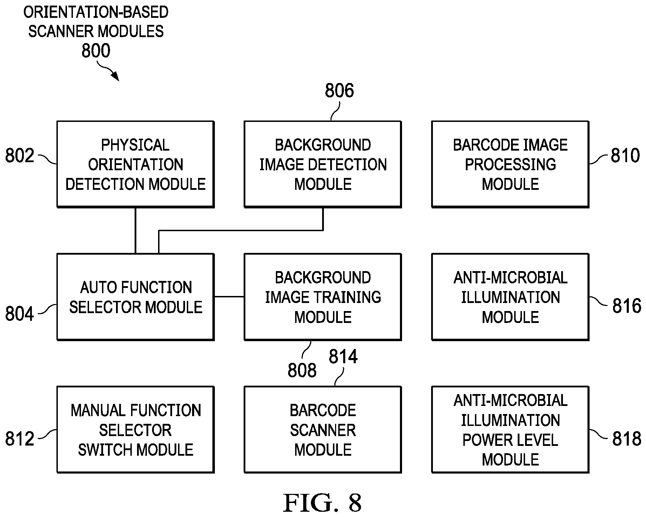

The processing unit **702** may include a single processor or multiple processors. The processing unit **702** may further include suitable logic, circuitry, and interfaces that are operable to execute one or more instructions **704,** such as, for example, modules **800** of **FIG. 8****,** based on sensor and other data received to perform operations of a scanner. The processing unit **702** may be realized through a number of processor technologies known in the art. The examples of the processing unit **702** may include, but are not limited to, an x86 processor, an ARM processor, a Reduced Instruction Set Computing (RISC) processor, an Application-Specific Integrated Circuit (ASIC) processor, an image processor, a digital signal processor, or a Complex Instruction Set Computing (CISC) processor. The I/O unit **706** may be configured to communicate data over a communications network (e.g., the Internet, wireless communications network, and so on).

The orientation-based scanner **714** may be any of the code readers described herein with reference to **FIGS. 1-6****.** The orientation-based scanner **714** may include a processing unit **716,** one or more orientation sensors **718,** an image sensor **720,** and memory **722.** The scanners **712** and orientation-based scanner **714** may communicate scan data **724** to the processing unit **702.** The scan data **724** may include data corresponding with an orientation of a housing of the orientation-based scanner **714.** The scan data **724** may include image data, code represented by a machine-readable indicia, or other data format that may be used or combined with other data by another processor operating on a host system (e.g., POS system), for example, based on a function in which the orientation-based scanner **714** is operating. It should be understood that the use of the housing as an orientation reference is arbitrary, and that any other object, such as a portion of a stand on which a barcode scanner is positioned, may be considered relative to the housing.

The orientations sensor(s) **718** may be any sensor that is capable of measuring angular or other position of the orientation-based scanner **710.** The image sensor **720** may include an optical camera and processing unit or otherwise (i) that enables a visual orientation of the orientation-based scanner **710** to be used in determining orientation as well as (ii) captures images to perform reading of machine-readable indicia or otherwise.

The code reader system **700** may also include an anti-microbial light source **726.** In one embodiment, the anti-microbial light source **726** may include a light emitting diode (LED) configured to emit an illumination having a wavelength between approximately 380 nanometers and approximately 470 nanometers, which are blue wavelengths. In an embodiment, the anti-microbial light source **726** may additionally and/or alternatively include an ultraviolet lighting elements (e.g., LEDs). It should be understood, however, that it has been found that ultraviolet light may be harmful to humans, so the use of ultraviolet light may be limited to times during which no humans are determined to be present through sensing (e.g., motion sensing, light sensing, notification from local security system, etc.), timing (e.g., between 1AM and 5AM), covering (e.g., shade pulled over workstation), or other means. The anti-microbial light source **726** may be used to clean or disinfect surfaces from unwanted microbes (i.e., deactivate or kill germs and/or bacteria). The anti-microbial light source **726** may be used to clean or disinfect the surfaces, which may include a workspace, touch points on a scanner and/or point-of-sale, etc., without cleaning chemicals that are often harmful to and/or degrade materials, such as polycarbonate and ABS, that are often used on data collection workspaces, on point-of-sale counters, on barcode scanners, or elsewhere in commercial settings at which barcode scanners are used.

With regard to **FIG. 8****,** a block diagram of modules **800** executed by a barcode reader system or barcode reader configured to automatically select and perform functions based on orientation and perform barcode reading using a selected function is shown. The modules **800** may include modules for detecting orientation and selecting a corresponding function, such as, but not limited to, a physical orientation detection module **802,** an automatic function selector module **804,** a background image detection module **806,** and a background image training module **808.** The physical orientation detection module **802** may include detecting an orientation and/or a change in orientation. The automatic function selector module **804** may be configured to select a function corresponding with an orientation detected by the physical orientation detection module **802.** In an embodiment, the number of available functions may be five, as described with regard to **FIGS. 2** and **3**. Other numbers of functions are also possible.

The background image detection module **806** may be configured to detect a background according to any of the hereinabove described background detection methods. The background image detection module **806** may be configured to search for and detect predetermined parameters of a background image obtained by an image sensor. The background image training module **808** may include capturing an image of a background when the barcode reader is in an orientation, and processing an indicator of a portion, or all, of an image captured by an image sensor. The image captured or derivation thereof may be stored in memory and a corresponding orientation may be assigned thereto. The background image training module **808** may include identifying parameters of a background image obtained by the image sensor that may be easily detected in subsequent scans of objects with the same background. For example, a rectangle in which checks are scanned may be used to define a recognizable pattern that is within a background portion of an image when the scanner is rotated or otherwise positioned to capture an image inclusive of the rectangle, thereby informing the module **804** of the barcode reader to change to a check reader function. Additionally and/or alternatively colors, shapes, or other visually distinguishing features on a surface that defines a background portion of an image of a target area in which objects may be scanned may be utilized to determine orientation via image training and image processing.

The modules **800** may also include a barcode image processing module **810,** a manual function selector switch module **812,** and a barcode scanner module **814.** In one embodiment, the manual function selector switch module **812** receives a communication, such as a signal from a switch or user interface, from a user in order to direct the processing to select a function identified in the user communication. The barcode scanner module **814** may include scanning a machine-readable indicia on an item in a field-of-view of a code reader inclusive of the modules **800.** The barcode scanner module **814** may further include capturing an image of a machine-readable indicia, or barcode, disposed on the item. The machine-readable-indicia may be a code representative of the item. The barcode image processing module **810** may include (i) receiving an image of a machine-readable indicia from the barcode scanner module **814** and (ii) processing the image to determine the code of the machine-readable indicia representative of the item.

The modules **800** may also include an anti-microbial illumination module **816** and an anti-microbial illumination power level module **818.** The anti-microbial illumination module **816** may be configured to initiate an emission of an anti-microbial illumination by the barcode scanner with anti-microbial illumination devices in response to the code reader being in an orientation corresponding with a cleaning function. The module **816** may additionally and/or alternatively be configured to initiate an emission of an anti-microbial illumination in response to other triggers or inputs, including a periodic (e.g., timer) or aperiodic (e.g., activation of a manual switch) event. In one embodiment, the anti-microbial illumination module **816** may receive an input that the code reader is in the orientation corresponding with the cleaning function. The anti-microbial illumination module **816** may be configured to stop an emission of the anti-microbial illumination in response to receiving an input that the code reader is configured in a non-anti-microbial illumination orientation. In one embodiment, the anti-microbial illumination module **816** may cause the anti-microbial illumination to be emitted by anti-microbial illumination device(s) when the code reader is powered on. In one embodiment, the anti-microbial illumination module **816** may cause the anti-microbial illumination to be emitted by the code reader after a single use or multiple uses of the code reader. In one embodiment, the anti-microbial illumination module **816** may cause the anti-microbial illumination to be emitted when a venue of the code reader is closed and/or unoccupied based on a timer, ambient lighting, audio sensing, motion sensing, and/or otherwise. It should be understood that the module **816** may be configured to turn on and off the anti-microbial illumination device(s) using a variety of other inputs that the module **816** uses to determine when to turn on and off the anti-microbial illumination device(s).

The anti-microbial illumination power level module **818** may be configured to control a power level of the anti-microbial illumination by anti-microbial illumination device(s). As an example, module **818** may be configured to cause the code reader to emit the anti-microbial illumination at a first power level when the code reader is in an orientation corresponding with a scanning function. The module **818** may further be configured to cause the code reader to emit the anti-microbial illumination at a second power level, which may be greater than the first power level, when the code reader is in an orientation corresponding with a cleaning or disinfection function.

The anti-microbial illumination power level module **818** may cause the anti-microbial illumination be emitted at either the first power level or the second power level in response to the code reader being in a corresponding orientation. For example, the code reader may be in a reading orientation, which may cause the module **816** to maintain the anti-microbial illumination device(s) to be off, in which case the module **818** does not set a power level of the anti-microbial illumination device(s), or be on and send an input (e.g., reader in reading mode) to the module **818** that drives the anti-microbial illumination device(s) to be in a first (low) illumination level. In an alternative embodiment, the module **818** may be configured to set an illumination level in response to periodic and/or aperiodic events described hereinabove with regard to the anti-microbial illumination module **816.** It should be understood that the module **818** may be configured to set power level of the anti-microbial illumination device(s) based on a variety of different rules or conditions. A retractable diffuser may be disposed in front of the anti-microbial illumination device(s) to distribute sanitizing light.

With regard to **FIG. 9****,** a flow diagram of a method **900** of automatically selecting a function of a code reader based on an orientation thereof is shown. The method **900** may include a step **902** of sensing a new orientation of a housing of the code reader. In sensing the new orientation, any of the hereinabove referenced sensing methods, such as, for example, sensing a new orientation of a housing of a code reader by an electromechanical sensor may be used.

The method **900** may then automatically change a function of the code reader based on the new orientation at step **904.** A processing unit of the code reader may access a database or table in a memory of corresponding functions and orientations. Based on the new orientation, the processing unit may select the corresponding function as listed in the database. In one embodiment, the processing unit may receive an orientation signal indicating that a new orientation has been sensed. The processing unit may respond to the orientation signal to identify the new orientation. In another embodiment, the new orientation may be identified in the orientation signal. For example, if a set of electrical contacts define different positions of the barcode reader, then the processing unit may switch a function corresponding to the identified orientation (e.g., 1 = barcode scanning, 2 = check scanning, etc.). At step **906,** the processing unit may update a portion of memory with the new orientation.

With regard to **FIG. 10****,** a flow diagram of a method **1000** of automatically selecting a function of a code reader based on an orientation thereof is shown. The method **1000** may begin with step **1002** when a scan is initiated. In one embodiment, the scan is initiated in response to a user communicating to a processing unit of a code reader to initiate a scan, such as, for example, squeezing a button or trigger on the code reader to initiate the scan. In another embodiment, the scan may be automatically initiated by identifying an item entering into a target area. The scan may be automatically initiated and the item sensed by at least one of electronics internal to the code reader and electronics external to the code reader.

At step **1004,** an orientation of the housing may be detected. In one embodiment, the code reader may include a sensor for sensing orientation of the housing. In another embodiment, the orientation may be detected by an image of a background of the target area. One of skill in the art will appreciate that many methods exist for detecting orientation, such as, but not limited, the methods of detecting orientation as described herein. At step **1006,** the processing unit may determine if the detected orientation is different than a current orientation as stored in memory. If the orientations are different, the processing unit may change a function of the code reader based on the detected orientation at step **1008.** In one embodiment, the processing unit may update the current orientation in memory with the detected orientation. At step **1010,** the code reader may scan the target area using a function corresponding to the detected orientation. The processing unit may direct components of the code reader to perform scanning functions corresponding with the detected orientation at step **1012.** The functions may be functions described hereinabove with reference to **FIGS. 1-6****.** The difference between the processes **900** and **1000** is the trigger as to when a function of the barcode reader is changed.

With regard to **FIGS. 11A** and **11B****,** illustrations of illustrative code readers **1100a** and **1100b** (collectively **1100**) which, according to the invention, are positioned in orientations corresponding with a cleaning function are shown. The code readers **1100** includes a first scanner **1102a** and a second scanner **1102b** (collectively **1102**) that are positioned on arms **1104a** and **1104b** (collectively **1104**) to enable the scanners **1102** to read machine-readable indicia on objects positioned. In addition to the scanners **1102,** the code reader **1100** may also include sideview scanners **1108a** and **1108b** (collectively **1108**) that are oriented to read machine-readable indicia positioned on objects when facing the scanner(s) **1108.** A bottom scanner **1110** may be positioned beneath the surface **1106** to scan machine-readable indicia facing downwards on the objects placed on the surface **1106.**

To initiate a disinfecting or cleaning function, the scanner(s) **1102** are positioned in an orientation corresponding with the cleaning function by rotating the arm(s) **1104** at joints **1112a** and **1112b** along the respective arms **1104.** The cleaning function are executed by turning on anti-microbial illumination devices (not shown) that illuminate fields-of-view **1114a** and **1114b** (collectively **1114**) of the scanners **1102.** The disinfecting function may disinfect surfaces within the fields-of-view **1114** of the scanner(s) **1102** that may include, but are not limited to, the surface **1106,** objects being scanned, user interface (e.g., keyboard), a housing of the scanner(s) **1102,** and/or any other surface or object in the fields-of-view **1114.** In one embodiment, the housing of the scanners **1102** may be substantially transparent so that the emitted anti-microbial illumination may clean the housing.

In one embodiment, the scanner(s) **1108** may also be configured with anti-microbial illumination device(s) to disinfect the surface **1106** and/or anything in fields-of-view of the scanners **1108.** The third scanner **1110** may also be configured with anti-microbial illumination device(s) to disinfect the surface **1106** and/or anything in the field-of-view of the scanner **1110.** One of skill in the art will appreciate that a number of combinations of anti-microbial illumination may be incorporated into the scanners **1102, 1108,** and **1110** or elsewhere at the code reader **1100.**

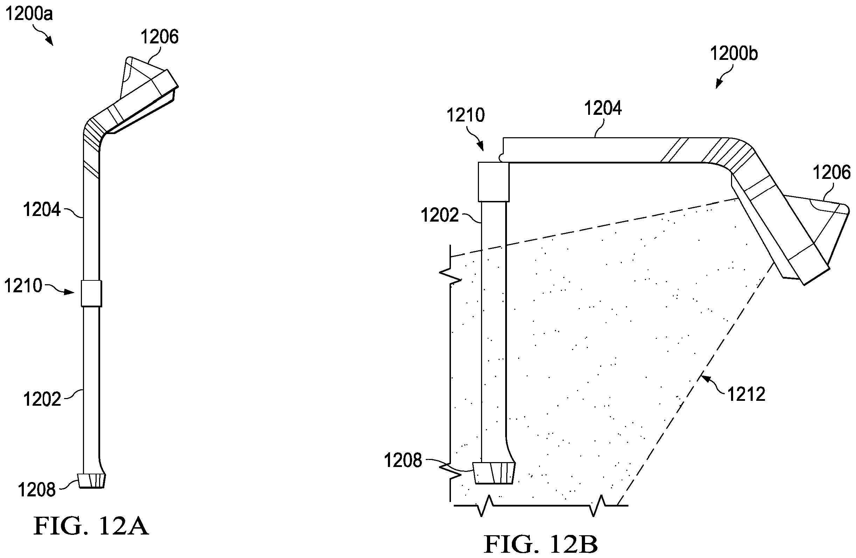

With regard to **FIGS. 12A** and **12B****,** illustrations of an illustrative top-down reader **1200a, 1200b** (collectively **1200**) which, according to the invention, which are inclusive of an anti-microbial illumination source configured to emit an anti-microbial illumination for cleaning or disinfecting are shown. The top-down reader **1200** may include a lower portion **1202,** an upper portion **1204,** and a scanner **1206.**

The top-down reader **1200** may be configured to extend upward from a housing of a code reader, such as the code reader **1100a** of **FIG. 11A****,** via an adapter **1208** that may be rotatable or otherwise enable a user to rotate the scanner **1206** relative to the code reader. In an embodiment, a joint **1210** between the upper portion **1204** and lower portion **1202** may be configured to enable the upper portion **1202** and lower portion **1204** to be axially rotatable relative to one another. In addition and/or alternatively, the joint **1210** may enable the upper portion to be rotated downwards toward the bottom portion **1202.** In one embodiment, sensors (not shown) may be disposed within or at the joint **1210,** such as the electromechanical sensors **508** of **FIG. 5****,** to sense axial rotation of the upper portion **1202** relative to the lower portion **1204.** Other sensor(s) (not shown) may be configured to detect vertical rotation of the upper portion **1202** relative to the lower portion **1204.** In an embodiment, the top-down reader **1200** may be configured to have at least two orientations that may include an upright orientation, such as the top-down reader **1200a** of **FIG. 12A****,** and a non-upright orientation, such as the top-down reader **1200b** of **FIG. 12B****.**

In one embodiment, the non-upright orientation of the top-down reader **1200b** may correspond with a cleaning function. The cleaning function may include the scanner **1206b** emitting an anti-microbial illumination into a field-of-view **1212** of the scanner **1206b.** In one embodiment, the top-down reader **1200** may be configured with a sensing device, such as a mercury switch, gyroscopes, accelerometers, or otherwise, to sense that the top-down reader **1200** is in an orientation corresponding with the cleaning function, and respond by executing the cleaning function (e.g., turning on anti-microbial illumination devices, and possibly at one of multiple possible illumination intensity levels). In one embodiment, a processing unit or static logic of the code reader **1200** may sense that the top-down reader **1200** is in the orientation corresponding with the cleaning function and respond by communicating a command to execute the cleaning function to the top-down reader **1200.**

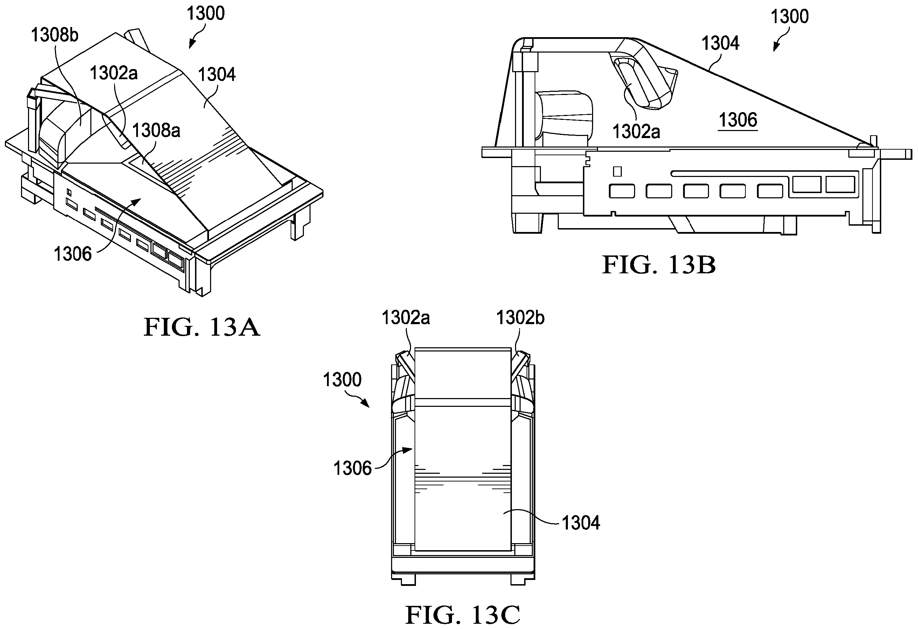

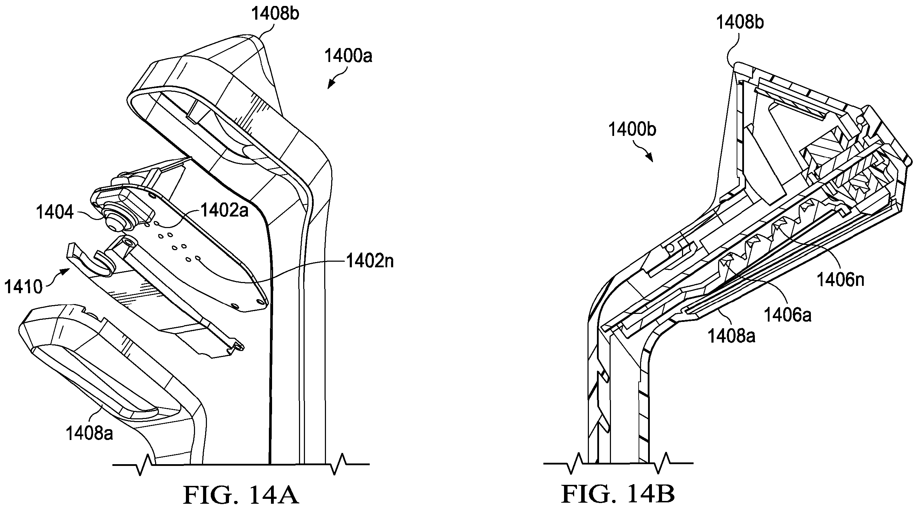

With regard to **FIGS. 13A-13C****,** illustrations of an illustrative code reader **1300** which, according to the invention, are inclusive of scanners **1302a** and **1302b** (collectively **1302**) and a reflective roller shade **1304** for use during a cleaning function are shown. The reflective roller shade **1304** may be configured to cover the scanners **1302** to create a covered workspace **1306.** The covered workspace **1306** may be substantially dark and provide a more efficient disinfecting condition for the cleaning function to kill germs and bacteria. In one embodiment, the code reader **1300** may include a sensor (see **FIG. 14A**) for sensing when the covered workspace is dark and in a condition for the cleaning function.

More specifically, the cleaning function includes the scanners **1302** emitting an anti-microbial illumination into the covered workspace **1306.** The anti-microbial illumination may reflect off of the reflective roller shade **1304** and clean additional portions of the code reader external to a field-of-view of the scanners **1302.**

In one embodiment, the code reader **1300** may include additional scanners **1308a** and **1308b** (collectively **1308**) that may also be configured to emit an additional anti-microbial illumination to the anti-microbial illumination emitted by the scanners **1302.** In one embodiment, the reflective roller shade **1304** may be configured to be retractable for ease of use by a user of the code reader **1300.** The roller shade **1304** may be manually positioned and retracted. In another embodiment, an event, such as a "clean" setting being selected via a user interface, may trigger (e.g., electronic command signal) the reflective roller shade **1304** to automatically deploy over the code reader **1300.**

With regard to **FIG. 14A** and **14B****,** illustrations of an illustrative scanner **1400a, 1400b** (collectively **1400**) inclusive of light sources **1402a-1402n** (collectively **1402**), and an image sensor **1404** configured to be used in cleaning functions and/or scanning functions are shown. The scanner **1400** may also include illumination lenses **1406a-1406n** (collectively **1406**) and housing members **1408a** and **1408b** (collectively **1408**). The scanner **1400** may be a scanner of a code reader, such as the scanners **1102, 1206,** and **1302** of **FIGS. 11A-13C****.**

The light sources **1402,** image sensor **1404,** and illumination lens **1406** may be disposed within the housing **1408.** The housing **1408** may include a lower portion **1408a** and an upper portion **1408b** that attach to one another. In one embodiment, the light sources **1402** and image sensor **1404** may be coupled to a cover **1410** for physical protection.

The light sources **1402** may include eight multi-color LEDs that correspond with eight illumination lenses **1406** to direct and shape an illumination field-of-view. The light source **1402** may be configured to emit a plurality of combinations of colors in response to different orientations of the code reader. In one embodiment of the code reader being in an orientation corresponding with a scanning function, the light source **1402** may emit (i) a red illumination only for performing reading of a machine-readable indicia, (ii) a combination of blue illumination and red illumination for disinfecting/reading, or (iii) any other combination of colors including or not including the blue illumination. The blue illumination may be an anti-microbial wavelength and/or ultraviolet wavelength. In one embodiment of the code reader being in an orientation corresponding with a cleaning function, the light source **1402** may emit only a blue illumination. Other colors during the cleaning function may be utilized, even for decorative purposes, so long as the anti-microbial functionality of killing harmful bacteria is not reduced. A power level of the anti-microbial illumination may be fixed, have multiple power levels, or be variable (e.g., change wavelengths and/or power levels over time). The power level may be set based on a setting or may be set by a manufacturer. As previously described, the level of the anti-microbial illumination may be set depending on a configuration, such as a cover being positioned for a cleaning mode, scanner with the anti-microbial light sources being placed into a particular position, setting be selected, or otherwise, of the code reader. If the barcode reader uses an image sensor, then the reader may be configured with anti-microbial illumination device(s) and, optionally, guide lights to assist a user with positioning a machine-readable indicia for scanning.

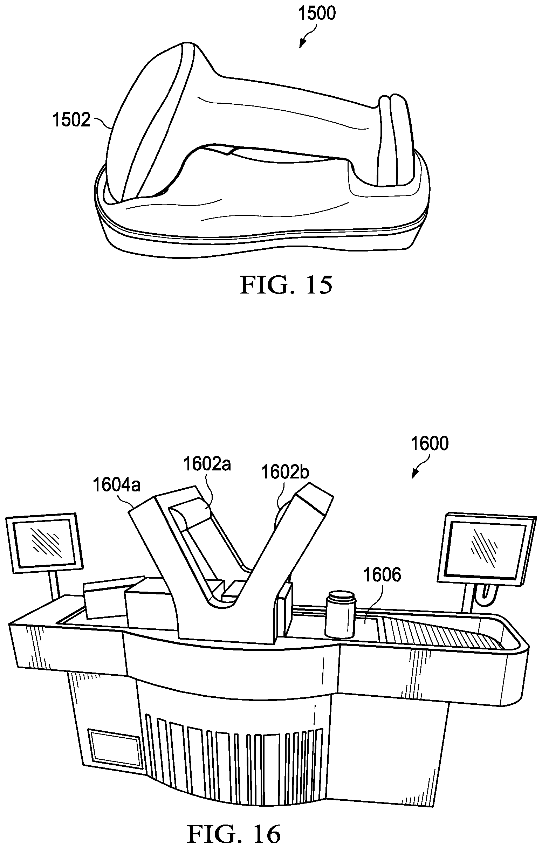

With regard to **FIG. 15****,** an illustration of an illustrative handheld code reader **1500** having a substantially transparent housing **1502** is shown. The handheld code reader **1500** may include a light source disposed within the housing **1502** and configured to emit an anti-microbial illumination over blue wavelengths, as previously described. In one embodiment, the light source may also be configured to scan an object. In one embodiment, the light source may be configured to emit the anti-microbial illumination outward to a portion of the housing **1502** that is held by a user and may be additional to a light source for scanning objects. One of skill in the art will appreciate that a plurality of combinations of light sources, including, but not limited to, one light source and two or more light sources, may be used for scanning, cleaning, and disinfecting. It should be understood that other, non-handheld code readers may include a portion or entire translucent or transparent housing to enable disinfecting the code readers in addition to surfaces, such as workspaces on which food and items are placed during purchasing or processing (e.g., performing inventory) operations.

With regard to **FIG. 16****,** an illustration of an illustrative code reader **1600** inclusive of a conveyor belt **1604** with overhead scanners **1602a** and **1602b** (collectively **1602**) positioned on arches **1604a** and **1604b** (collectively **1604**) having a cleaning function is shown. In one embodiment, the overhead scanner **1602** may include a single arch including at least one scanner. In addition to the overhead scanners **1602** positioned on the arches, additional scanners may be positioned prior to, after, and beneath the arches **1604.** The overhead scanners **1602,** as well as any of the other scanners, may be configured with anti-microbial illumination sources to emit an anti-microbial illumination for cleaning and/or disinfecting the conveyor belt **1606.** Additionally and/or alternatively, anti-microbial illumination devices may be positioned separate from the scanner and disposed inside of or external from the arches

In one embodiment, the overhead scanners **1602** and any other scanner that may be configured with anti-microbial illumination source(s) may be configured to clean and/or disinfect the conveyor belt **1606** and/or other work surfaces in response to any number of triggers, as previously described. For example, the overhead scanner **1602** may be configured to perform a cleaning and/or disinfecting function at a regular time interval (e.g., at 3AM) and for a predetermined duration (e.g., 30 minutes). The overhead scanner **1602** may also be configured to perform the cleaning and/or disinfecting function in response to a command from a user. The command from the user may be active (i.e., flipping a switch or pushing a button) or passive (i.e., shutting down the code reader **1600,** performing an action or repositioning a component of the code reader **1600** to indicate that the code reader **1600** is done being used for a period of time).

In one embodiment, the overhead scanner **1602** may be configured to perform the cleaning and/or disinfecting function for a set length of time. The overhead scanner **1602** may be configured to perform the cleaning and/or disinfecting function for a set number of rotations of the conveyor belt **1606,** such as 20 rotations). In operation, the conveyor belt **1606** may be controllable by a processing unit of the scanning system (e.g., a processing unit that is communication with each of the scanners at the code reader **1600**) to turn on and off the conveyor belt **1606** during times that the cleaning function is being performed and controlling speed of the conveyor belt **1606** (e.g., slower than normal speed during a cleaning mode to enable higher durations of time of being exposed to the anti-microbial illumination). One of skill in the art will appreciate that many methods of initiating and timing a cleaning function may exist for operating the overhead scanner **1602.**

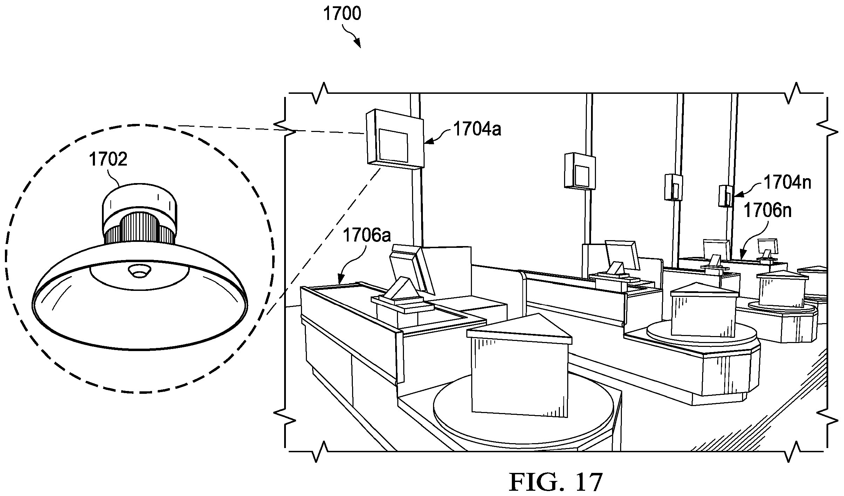

With regard to **FIG. 17****,** an illustration of an illustrative code reader station **1700** with an overhead anti-microbial illumination source **1702** for disinfecting a workspace of the station is shown. The code reader station **1700** may include the overhead anti-microbial illumination source **1702** disposed in a housings **1704a** - **1704n** (collectively **1704**) that is configured to hang or are supported by a post above workstations **1706a-1706n** (collectively **1706**) such that the overhead anti-microbial illumination source **1702** may flood the workstation **1706** with anti-microbial illumination.

In one embodiment, the overhead anti-microbial illumination source **1702** may emit an anti-microbial illumination. The overhead anti-microbial illumination source **1702** may be configured to emit the anti-microbial illumination in response to at least one of actions, orientations, and other commands as described hereinabove with regard to **FIGS. 11A-16****.** For example, the anti-microbial illumination source **1702** may be turned on in response to a scanner arm or other feature of the scanner being placed into a clean orientation. Other triggers may be utilized and illumination intensity may be set based on a variety of possible mechanisms, including motion sensing, light sensing, time of day, or any other sensing mechanism that enables a determination that no one is in a field-of-view of the anti-microbial illumination source **1702.** The motion, light, and other sensing may be considered sensing environmental factors. In an embodiment out of the invention, a first sense value (e.g., motion sensed, bright light sensed, etc.) that indicates that people may be local to the code reader may be used by a processor to maintain the anti-microbial illumination source **1702** at a first level, such as off or at a low power level, and a second sense value (e.g., no motion, darkness or low light sensed, etc.) that indicates that no one is local to the code reader may be used by the processor to turn on or increase the power level of the anti-microbial illumination source **1702.**

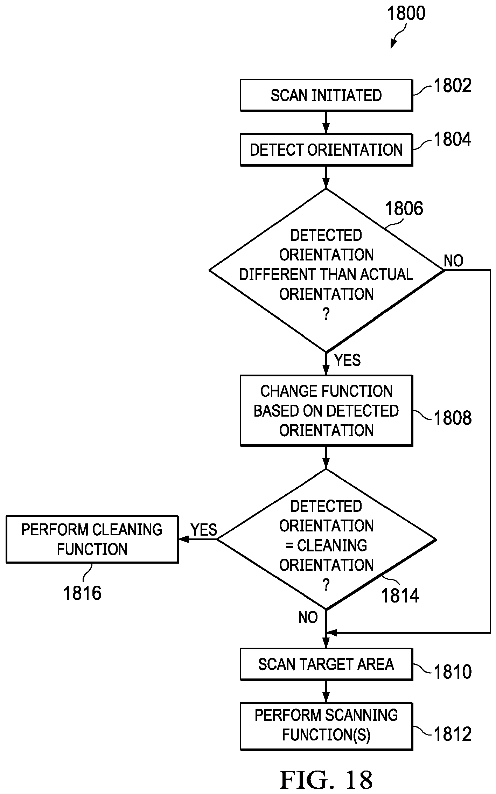

With regard to **FIG. 18****,** a flow diagram of an illustrative method **1800** of automatically selecting a function of a code reader based on an orientation thereof is shown. The method **1800** may begin with step **1802** when a scan is initiated in the same or similar manner as **FIG. 10****.** In one embodiment, the scan is initiated in response to a user communicating to a processing unit of a code reader to initiate a scan, such as, for example, squeezing a button or trigger on the code reader to initiate the scan. In another embodiment, the scan may be automatically initiated by identifying an item entering into a target area. The scan may be automatically initiated and the item sensed by at least one of electronics internal to the code reader and electronics external to the code reader.

At step **1804,** an orientation of the housing may be detected. In one embodiment, the code reader may include a sensor for sensing orientation of the housing. In another embodiment, the orientation may be detected by an image of a background of the target area. One of skill in the art will appreciate that many methods exist for detecting orientation, such as, but not limited, the methods of detecting orientation as described herein. At step **1806,** the processing unit may determine if the detected orientation is different than a current orientation as stored in memory. If the orientations are different, the processing unit may change a function of the code reader based on the detected orientation at step **1808.** In determining orientations, the orientation may include physical position of a scanner with anti-microbial device(s), support member of the scanner, adjustable member (e.g., cover) of the scanner, or otherwise. In an embodiment, in addition to determining orientation of the physical position, a determination of a selectable switch, button, or other setting mechanism may be determined. For example, a timer switch that is set to turn on for an hour a night when a store or other venue is closed may cause the cleaner function to be initiated. In one embodiment, the processing unit may update the current orientation in memory with the detected orientation (or other setting). At step **1810,** the code reader may scan the target area using a function corresponding to the detected orientation. The processing unit may direct components of the code reader to perform scanning functions corresponding with the detected orientation at step **1812.** The functions may be functions described hereinabove with reference to **FIGS. 1-6**.

At step **1814,** the processing unit determines if the detected orientation corresponds with a cleaning function. If the orientation corresponds with the cleaning function, the processing unit communicates to perform the cleaning function by emitting an anti-microbial illumination at step **1816.** If the orientation does not correspond with the cleaning function, the code reader scans the target area at step **1810** as described hereinabove. In an embodiment, the scanning and cleaning functions may be performed simultaneously.

One embodiment of the invention, the method for disinfecting a target area at the code reader includes determining if an orientation of a housing inclusive of a scanner for reading machine-readable indicia is an orientation corresponding with a cleaning function. In response to determining that the orientation of the housing is the orientation corresponding with the cleaning function, a cleaning function may be selected to cause an anti-microbial illumination to be emitted toward a target area of the scanner.

The anti-microbial illumination may emit a blue light having a wavelength between approximately 380 nanometers and approximately 470 nanometers. A determination if the orientation of the housing is the orientation corresponding with the cleaning function may include sensing a change in angular orientation of the housing. The housing is supported by an adjustable arm having a first position for enabling the scanner to scan machine-readable indicia and a second position associated with the cleaning function.

A first level of anti-microbial illumination may be produced in response to determining that the housing is in an orientation not associated with the cleaning function. A second, higher level of anti-microbial illumination may be produced in response to determining that the housing is in the orientation corresponding with the cleaning function. The anti-microbial illumination may be produced in combination with the red light.

A shade configured to cover the target area and source of the anti-microbial illumination is deployed may be sensed, and the anti-microbial illumination may be caused to be at a power level higher than when the anti-microbial illumination is used for reading machine-readable indicia. A signal may be utilized to emit an anti-microbial light in response to deploy the reflective roller shade.

One embodiment of a method out of the invention for disinfecting a surface at a code reader may include sensing an environmental factor at the code reader. In response to determining that the environmental factor is at a first value, the code reader may be configured to read machine-readable indicia. In response to determining that environmental factor is at a second value, an anti-microbial illumination may be caused to be emitted toward a target area of the scanner. In response to determining that the environmental factor is at the first value, the anti-microbial illumination may be caused to be emitted at a first power level. In response to determining that the environmental factor is at the second value, the anti-microbial illumination may be increased to be emitted at a second, higher power level.

The foregoing method descriptions and the process flow diagrams are provided merely as illustrative examples and are not intended to require or imply that the steps of the various embodiments must be performed in the order presented. As will be appreciated by one of skill in the art, the steps in the foregoing embodiments may be performed in any order. Words such as "then," "next," etc. are not intended to limit the order of the steps; these words are simply used to guide the reader through the description of the methods. Although process flow diagrams may describe the operations as a sequential process, many of the operations may be performed in parallel or concurrently. In addition, the order of the operations may be rearranged. A process may correspond to a method, a function, a procedure, a subroutine, a subprogram, etc. When a process corresponds to a function, its termination may correspond to a return of the function to the calling function or the main function.

The various illustrative logical blocks, modules, circuits, and algorithm steps described in connection with the embodiments disclosed here may be implemented as electronic hardware, computer software, or combinations of both. To clearly illustrate this interchangeability of hardware and software, various illustrative components, blocks, modules, circuits, and steps have been described above generally in terms of their functionality. Whether such functionality is implemented as hardware or software depends upon the particular application and design constraints imposed on the overall system. Skilled artisans may implement the described functionality in varying ways for each particular application, but such implementation decisions should not be interpreted as causing a departure from the scope of the present invention.

Embodiments implemented in computer software may be implemented in software, firmware, middleware, microcode, hardware description languages, or any combination thereof. A code segment or machine-executable instructions may represent a procedure, a function, a subprogram, a program, a routine, a subroutine, a module, a software package, a class, or any combination of instructions, data structures, or program statements. A code segment may be coupled to and/or in communication with another code segment or a hardware circuit by passing and/or receiving information, data, arguments, parameters, or memory contents. Information, arguments, parameters, data, etc. may be passed, forwarded, or transmitted via any suitable means including memory sharing, message passing, token passing, network transmission, etc.

The actual software code or specialized control hardware used to implement these systems and methods is not limiting of the invention. Thus, the operation and behavior of the systems and methods were described without reference to the specific software code being understood that software and control hardware can be designed to implement the systems and methods based on the description here.

When implemented in software, the functions may be stored as one or more instructions or code on a non-transitory computer-readable or processor-readable storage medium. The steps of a method or algorithm disclosed here may be embodied in a processor-executable software module which may reside on a computer-readable or processor-readable storage medium. A non-transitory computer-readable or processor-readable media includes both computer storage media and tangible storage media that facilitate transfer of a computer program from one place to another. A non-transitory processor-readable storage media may be any available media that may be accessed by a computer. By way of example, and not limitation, such non-transitory processor-readable media may comprise RAM, ROM, EEPROM, CD-ROM or other optical disk storage, magnetic disk storage or other magnetic storage devices, or any other tangible storage medium that may be used to store desired program code in the form of instructions or data structures and that may be accessed by a computer or processor. Disk and disc, as used here, include compact disc (CD), laser disc, optical disc, digital versatile disc (DVD), floppy disk, and Blu-ray disc where disks usually reproduce data magnetically, while discs reproduce data optically with lasers. Combinations of the above should also be included within the scope of computer-readable media. Additionally, the operations of a method or algorithm may reside as one or any combination or set of codes and/or instructions on a non-transitory processor-readable medium and/or computer-readable medium, which may be incorporated into a computer program product.

The previous description is of a preferred embodiment for implementing the invention, and the scope of the invention should not necessarily be limited by this description. The scope of the present invention is instead defined by the following claims.

## Claims

1. A code reader (1100, 1100a, 1100b, 1200, 1200a, 1200b, 1300), comprising:
a housing configured to be adjustably oriented, said housing including at least one orientation corresponding with a cleaning function;
a scanner (1102, 1102a, 1102b, 1108, 1108a, 1108b, 1110, 1206, 1206b, 1302, 1302a, 1302b, 1308, 1308a, 1308b, 1400, 1400a, 1400b) disposed within said housing, and configured to scan a machine-readable indicia in a target area;
an illumination source (1402, 1402a, 1402n) configured to emit an anti-microbial illumination; and
a processing unit in communication with said scanner (1102, 1102a, 1102b, 1108, 1108a, 1108b, 1110, 1206, 1206b, 1302, 1302a, 1302b, 1308, 1308a, 1308b, 1400, 1400a, 1400b) and said illumination source (1402, 1402a, 1402n), and configured to select the cleaning function to cause said illumination source (1402, 1402a, 1402n) to emit the anti-microbial illumination toward the target area to disinfect surfaces in the target area;
wherein said housing is further configured to be supported by an adjustable arm (1104, 1104a, 1104b) having a first position for enabling said scanner (1102, 1102a, 1102b, 1108, 1108a, 1108b, 1110, 1206, 1206b, 1302, 1302a, 1302b, 1308, 1308a, 1308b, 1400, 1400a, 1400b) to scan machine-readable indicia and a second position associated with the cleaning function, said processing unit being configured (i) to determine that the adjustable arm (1104, 1104a, 1104b) is in the second position, and (ii) to cause said anti-microbial illumination source (1402, 1402a, 1402n) to illuminate.

2. The code reader (1100, 1100a, 1100b, 1200, 1200a, 1200b, 1300) according to claim 1, wherein said scanner (1102, 1102a, 1102b, 1108, 1108a, 1108b, 1110, 1206, 1206b, 1302, 1302a, 1302b, 1308, 1308a, 1308b, 1400, 1400a, 1400b) is configured to capture an image of the target area, and wherein said processing unit is further configured to determine the orientation of said housing based on the image of the target area.

3. The code reader (1100, 1100a, 1100b, 1200, 1200a, 1200b, 1300) according to claim 2, wherein said processing unit, in determining the orientation, is configured to identify known features disposed in a background portion of the image of the target area.

4. The code reader (1100, 1100a, 1100b, 1200, 1200a, 1200b, 1300) according to claim 1, wherein the anti-microbial illumination includes at least one of a blue light and an ultraviolet light and preferably the blue light includes a light emitting diode that emits light at a wavelength in a range between approximately 380 nanometers and approximately 470 nanometers.

5. The code reader (1100, 1100a, 1100b, 1200, 1200a, 1200b, 1300) according to claim 4, wherein said processing unit is further configured to emit the anti-microbial illumination in an asynchronous pattern.

6. The code reader (1100, 1100a, 1100b, 1200, 1200a, 1200b, 1300) according to claim 1, wherein said processing unit is further configured to emit the anti-microbial illumination in combination with a red light.

7. The code reader (1100, 1100a, 1100b, 1200, 1200a, 1200b, 1300) according to claim 1, further comprising a reflective roller shade (1304) configured to be positioned in a retracted position and a deployed position, the deployed position configured to cover the target area and illumination source (1402, 1402a, 1402n).

8. The code reader (1100, 1100a, 1100b, 1200, 1200a, 1200b, 1300) according to claim 7, wherein said processing unit is further configured to receive a signal that the reflective roller shade (1304) is in the deployed position, and communicate a signal to said illumination source (1402, 1402a, 1402n) to emit the anti-microbial illumination in response to said reflective roller shade (1304) being in a deployed position.

9. A method for disinfecting a target area at a code reader (1100, 1100a, 1100b, 1200, 1200a, 1200b, 1300), comprising:
determining if an orientation of a housing inclusive of a scanner (1102, 1 102a, 1102b, 1108, 1108a, 1108b, 1110, 1206, 1206b, 1302, 1302a, 1302b, 1308, 1308a, 1308b, 1400, 1400a, 1400b) for reading machine-readable indicia is an orientation corresponding with a cleaning function, said housing being configured to be supported by an adjustable arm (1104, 1104a, 1104b) having a first position for enabling said scanner (1102, 1102a, 1102b, 1108, 1108a, 1108b, 1110, 1206, 1206b, 1302, 1302a, 1302b, 1308, 1308a, 1308b, 1400, 1400a, 1400b) to scan machine-readable indicia and a second position associated with the cleaning function; and
in response to determining that the orientation of the housing is the orientation corresponding with the cleaning function, selecting a cleaning function to cause an anti-microbial illumination to be emitted toward a target area of the scanner (1102, 1102a, 1102b, 1108, 1108a, 1108b, 1110, 1206, 1206b, 1302, 1302a, 1302b, 1308, 1308a, 1308b, 1400, 1400a, 1400b).

10. The method according to claim 9, wherein determining if the orientation of the housing is the orientation corresponding with the cleaning function includes sensing a change in angular orientation of the housing.

11. The method according to claim 9, further comprising:
producing a first level of anti-microbial illumination in response to determining that the housing is in an orientation not associated with the cleaning function; and
producing a second, higher level of anti-microbial illumination in response to determining that the housing is in the orientation corresponding with the cleaning function.

12. The method according to claim 9, further comprising:
sensing that a shade configured to cover the target area and source (1402, 1402a, 1402n) of the anti-microbial illumination is deployed; and
causing the anti-microbial illumination to be at a power level higher than when the anti-microbial illumination is used for reading machine-readable indicia.

13. The method according to claim 12, further comprising communicating a signal to all image sensors and scanners (1102, 1102a, 1102b, 1108, 1108a, 1108b, 1110, 1206, 1206b, 1302, 1302a, 1302b, 1308, 1308a, 1308b, 1400, 1400a, 1400b) with anti-microbial illumination sources (1402, 1402a, 1402n) to emit the anti-microbial illumination in response to deploying the reflective roller shade (1304).

## Patentansprüche

1. Codeleser (1100, 1100a, 1100b, 1200, 1200a, 1200b, 1300), umfassend:
ein Gehäuse, das dazu konfiguriert ist, einstellbar ausgerichtet zu sein, wobei das Gehäuse mindestens eine Ausrichtung enthält, die einer Reinigungsfunktion entspricht;
einen Scanner (1102, 1102a, 1102b, 1108, 1108a, 1108b, 1110, 1206, 1206b, 1302, 1302a, 1302b, 1308, 1308a, 1308b, 1400, 1400a, 1400b), der innerhalb des Gehäuses angeordnet ist und konfiguriert ist, um eine maschinenlesbare Angabe in einem Zielbereich abzutasten;
eine Beleuchtungsquelle (1402, 1402a, 1402n), die so konfiguriert ist, dass sie eine antimikrobielle Beleuchtung emittiert; und
eine Verarbeitungseinheit in Kommunikation mit dem Scanner (1102, 1102a, 1102b, 1108, 1108a, 1108b, 1110, 1206, 1206b, 1302, 1302a, 1302b, 1308, 1308a, 1308b, 1400, 1400a, 1400b) und der Beleuchtungsquelle (1402, 1402a, 1402n) und konfiguriert, um die Reinigungsfunktion auszuwählen, um zu bewirken, dass die Beleuchtungsquelle (1402, 1402a, 1402n) die antimikrobielle Beleuchtung in Richtung des Zielbereichs emittiert, um Oberflächen im Zielbereich zu desinfizieren;
wobei das Gehäuse ferner konfiguriert ist, um von einem einstellbaren Arm (1104, 1104a, 1104b) getragen zu werden, der eine erste Position, um es dem Scanner (1102, 1102a, 1102b, 1108, 1108a, 1108b, 1110, 1206, 1206b, 1302, 1302a, 1302b, 1308, 1308a, 1308b, 1400, 1400a, 1400b) zu ermöglichen, maschinenlesbare Angaben abzutasten, und eine zweite Position aufweist, die mit der Reinigungsfunktion assoziiert ist, wobei die Verarbeitungseinheit konfiguriert ist (i), um zu bestimmen, dass sich der einstellbare Arm (1104, 1104a, 1104b) in der zweiten Position befindet, und (ii), um zu bewirken, dass die antimikrobielle Beleuchtungsquelle (1402, 1402a, 1402n) leuchtet.

2. Codeleser (1100, 1100a, 1100b, 1200, 1200a, 1200b, 1300) nach Anspruch 1, wobei der Scanner (1102, 1102a, 1102b, 1108, 1108a, 1108b, 1110, 1206, 1206b, 1302, 1302a, 1302b, 1308, 1308a, 1308b, 1400, 1400a, 1400b) konfiguriert ist, um ein Bild des Zielbereichs aufzunehmen, und wobei die Verarbeitungseinheit ferner konfiguriert ist, um die Ausrichtung des Gehäuses basierend auf dem Bild des Zielbereichs zu bestimmen.

3. Codeleser (1100, 1100a, 1100b, 1200, 1200a, 1200b, 1300) nach Anspruch 2, wobei die Verarbeitungseinheit beim Bestimmen der Ausrichtung konfiguriert ist, um bekannte Merkmale zu identifizieren, die in einem Hintergrundabschnitt des Bildes des Zielbereichs angeordnet sind.

4. Codeleser (1100, 1100a, 1100b, 1200, 1200a, 1200b, 1300) nach Anspruch 1, wobei die antimikrobielle Beleuchtung mindestens entweder ein blaues Licht oder ein ultraviolettes Licht enthält und vorzugsweise das blaue Licht eine lichtemittierende Diode enthält, die Licht mit einer Wellenlänge in einem Bereich zwischen ungefähr 380 Nanometern und ungefähr 470 Nanometern emittiert.

5. Codeleser (1100, 1100a, 1100b, 1200, 1200a, 1200b, 1300) nach Anspruch 4, wobei die Verarbeitungseinheit ferner so konfiguriert ist, dass sie die antimikrobielle Beleuchtung in einem asynchronen Muster emittiert.

6. Codeleser (1100, 1100a, 1100b, 1200, 1200a, 1200b, 1300) nach Anspruch 1, wobei die Verarbeitungseinheit ferner so konfiguriert ist, dass sie die antimikrobielle Beleuchtung in Kombination mit einem roten Licht emittiert.

7. Codeleser (1100, 1100a, 1100b, 1200, 1200a, 1200b, 1300) nach Anspruch 1, ferner umfassend ein reflektierendes Rollo (1304), das so konfiguriert ist, dass es in einer eingefahrenen Position und einer ausgefahrenen Position positioniert werden kann, wobei die ausgefahrene Position so konfiguriert ist, dass sie den Zielbereich und die Beleuchtungsquelle (1402, 1402a, 1402n) abdeckt.

8. Codeleser (1100, 1100a, 1100b, 1200, 1200a, 1200b, 1300) nach Anspruch 7, wobei die Verarbeitungseinheit ferner konfiguriert ist, um ein Signal zu empfangen, dass sich das reflektierende Rollo (1304) in der ausgefahrenen Position befindet, und ein Signal an die Beleuchtungsquelle (1402, 1402a, 1402n) zu übermitteln, um die antimikrobielle Beleuchtung in Reaktion darauf zu emittieren, dass sich das reflektierende Rollo (1304) in einer ausgefahrenen Position befindet.

9. Verfahren zum Desinfizieren eines Zielbereichs an einem Codeleser (1100, 1100a, 1100b, 1200, 1200a, 1200b, 1300), umfassend:
Bestimmen, ob eine Ausrichtung eines Gehäuses einschließlich eines Scanners (1102, 1102a, 1102b, 1108, 1108a, 1108b, 1110, 1206, 1206b, 1302, 1302a, 1302b, 1308, 1308a, 1308b, 1400, 1400a, 1400b) zum Lesen von maschinenlesbaren Angaben eine Ausrichtung ist, die einer Reinigungsfunktion entspricht, wobei das Gehäuse konfiguriert ist, um von einem einstellbaren Arm (1104, 1104a, 1104b) getragen zu werden, der eine erste Position, um es dem Scanner (1102, 1102a, 1102b, 1108, 1108a, 1108b, 1110, 1206, 1206b, 1302, 1302a, 1302b, 1308, 1308a, 1308b, 1400, 1400a, 1400b) zu ermöglichen, maschinenlesbare Angaben abzutasten, und eine zweite Position aufweist, die mit der Reinigungsfunktion assoziiert ist; und
als Reaktion auf die Bestimmung, dass die Ausrichtung des Gehäuses die Ausrichtung ist, die der Reinigungsfunktion entspricht, Auswählen einer Reinigungsfunktion, um zu bewirken, dass eine antimikrobielle Beleuchtung in Richtung eines Zielbereichs des Scanners (1102, 1102a, 1102b, 1108, 1108a, 1108b, 1110, 1206, 1206b, 1302, 1302a, 1302b, 1308, 1308a, 1308b, 1400, 1400a, 1400b) emittiert wird.

10. Verfahren nach Anspruch 9, wobei das Bestimmen, ob die Ausrichtung des Gehäuses die Ausrichtung ist, die der Reinigungsfunktion entspricht, das Erfassen einer Änderung der Winkelausrichtung des Gehäuses enthält.

11. Verfahren nach Anspruch 9, ferner umfassend:
Erzeugen einer ersten Ebene antimikrobieller Beleuchtung als Reaktion auf die Bestimmung, dass sich das Gehäuse in einer Ausrichtung befindet, die nicht mit der Reinigungsfunktion assoziiert ist; und
Erzeugen einer zweiten, höheren Ebene antimikrobieller Beleuchtung als Reaktion auf die Bestimmung, dass sich das Gehäuse in der Ausrichtung befindet, die der Reinigungsfunktion entspricht.

12. Verfahren nach Anspruch 9, ferner umfassend:
Erfassen, dass ein Rollo, das konfiguriert ist, um den Zielbereich und die Quelle (1402, 1402a, 1402n) der antimikrobiellen Beleuchtung abzudecken, ausgefahren ist; und
Veranlassen, dass die antimikrobielle Beleuchtung auf einer höheren Leistungsebene ist, als wenn die antimikrobielle Beleuchtung zum Lesen von maschinenlesbaren Angaben verwendet wird.

13. Verfahren nach Anspruch 12, ferner umfassend das Übermitteln eines Signals an alle Bildsensoren und Scanner (1102, 1102a, 1102b, 1108, 1108a, 1108b, 1110, 1206, 1206b, 1302, 1302a, 1302b, 1308, 1308a, 1308b, 1400, 1400a, 1400b) mit antimikrobiellen Beleuchtungsquellen (1402, 1402a, 1402n) zum Emittieren der antimikrobiellen Beleuchtung als Reaktion auf das Ausfahren des reflektierenden Rollos (1304).

## Revendications

1. Lecteur de code à barres (1100, 1100a, 1100b, 1200, 1200a, 1200b, 1300), comprenant :
un boîtier configuré pour être orienté de manière réglable, ledit boîtier comprenant au moins une orientation correspondant à une fonction de nettoyage ;
un scanner (1102, 1102a, 1102b, 1108, 1108a, 1108b, 1110, 1206, 1206b, 1302, 1302a, 1302b, 1308, 1308a, 1308b, 1400, 1400a, 1400b) disposé à l'intérieur dudit boîtier, et configuré pour balayer des indices lisibles par machine dans une zone cible ;
une source d'éclairage (1402, 1402a, 1402n) configurée pour émettre un éclairage antimicrobien ; et
une unité de traitement en communication avec ledit scanner (1102, 1102a, 1102b, 1108, 1108a, 1108b, 1110, 1206, 1206b, 1302, 1302a, 1302b, 1308, 1308a, 1308b, 1400, 1400a, 1400b) et ladite source d'éclairage (1402, 1402a, 1402n), et configurée pour sélectionner la fonction de nettoyage pour amener ladite source d'éclairage (1402, 1402a, 1402n) à émettre l'éclairage antimicrobien vers la zone cible pour désinfecter des surfaces dans la zone cible ;
dans lequel ledit boîtier est en outre configuré pour être supporté par un bras réglable (1104, 1104a, 1104b) ayant une première position pour permettre audit scanner (1102, 1102a, 1102b, 1108, 1108a, 1108b, 1110, 1206, 1206b, 1302, 1302a, 1302b, 1308, 1308a, 1308b, 1400, 1400a, 1400b) de balayer des indices lisibles par machine et une seconde position associée à la fonction de nettoyage, ladite unité de traitement étant configurée (i) pour déterminer que le bras réglable (1104, 1104a, 1104b) est dans la seconde position, et (ii) pour faire en sorte que ladite source d'éclairage antimicrobien (1402, 1402a, 1402n) s'allume.

2. Lecteur de code à barres (1100, 1100a, 1100b, 1200, 1200a, 1200b, 1300) selon la revendication 1, dans lequel ledit scanner (1102, 1102a, 1102b, 1108, 1108a, 1108b, 1110, 1206, 1206b, 1302, 1302a, 1302b, 1308, 1308a, 1308b, 1400, 1400a, 1400b) est configuré pour capturer une image de la zone cible, et dans lequel ladite unité de traitement est en outre configurée pour déterminer l'orientation dudit boîtier sur la base de l'image de la zone cible.

3. Lecteur de code à barres (1100, 1100a, 1100b, 1200, 1200a, 1200b, 1300) selon la revendication 2, dans lequel ladite unité de traitement, pour déterminer l'orientation, est configurée pour identifier des caractéristiques connues disposées dans une partie de fond de l'image de la zone cible.

4. Lecteur de code à barres (1100, 1100a, 1100b, 1200, 1200a, 1200b, 1300) selon la revendication 1, dans lequel l'éclairage antimicrobien comprend au moins une lumière bleue et une lumière ultraviolette et de préférence la lumière bleue comprend une diode électroluminescente qui émet de la lumière à une longueur d'onde dans une plage comprise entre environ 380 nanomètres et environ 470 nanomètres.

5. Lecteur de code à barres (1100, 1100a, 1100b, 1200, 1200a, 1200b, 1300) selon la revendication 4, dans lequel ladite unité de traitement est en outre configurée pour émettre l'éclairage antimicrobien selon un motif asynchrone.

6. Lecteur de code à barres (1100, 1100a, 1100b, 1200, 1200a, 1200b, 1300) selon la revendication 1, dans lequel ladite unité de traitement est en outre configurée pour émettre l'éclairage antimicrobien en combinaison avec une lumière rouge.

7. Lecteur de code à barres (1100, 1100a, 1100b, 1200, 1200a, 1200b, 1300) selon la revendication 1, comprenant en outre un store à rouleau réfléchissant (1304) configuré pour être positionné dans une position rétractée et une position déployée, la position déployée étant configurée pour couvrir la zone cible et la source d'éclairage (1402, 1402a, 1402n).

8. Lecteur de code à barres (1100, 1100a, 1100b, 1200, 1200a, 1200b, 1300) selon la revendication 7, dans lequel ladite unité de traitement est en outre configurée pour recevoir un signal indiquant que le store à rouleau réfléchissant (1304) est dans la position déployée, et communiquer un signal à ladite source d'éclairage (1402, 1402a, 1402n) pour émettre l'éclairage antimicrobien en réponse au fait que ledit store à rouleau réfléchissant (1304) est dans une position déployée.

9. Procédé de désinfection d'une zone cible sur un lecteur de codes à barres (1100, 1100a, 1100b, 1200, 1200a, 1200b, 1300), comprenant :
la détermination de si une orientation d'un boîtier comprenant un scanner (1102, 1102a, 1102b, 1108, 1108a, 1108b, 1110, 1206, 1206b, 1302, 1302a, 1302b, 1308, 1308a, 1308b, 1400, 1400a, 1400b) pour lire des indices lisibles par machine est une orientation correspondant à une fonction de nettoyage, ledit boîtier étant configuré pour être supporté par un bras réglable (1104, 1104a, 1104b) ayant une première position pour permettre audit scanner (1102, 1102a, 1102b, 1108, 1108a, 1108b, 1110, 1206, 1206b, 1302, 1302a, 1302b, 1308, 1308a, 1308b, 1400, 1400a, 1400b) de balayer des indices lisibles par machine et une seconde position associée à la fonction de nettoyage ; et
en réponse à la détermination du fait que l'orientation du boîtier est l'orientation correspondant à la fonction de nettoyage, la sélection d'une fonction de nettoyage pour provoquer l'émission d'un éclairage antimicrobien vers une zone cible du scanner (1102, 1102a, 1102b, 1108, 1108a, 1108b, 1110, 1206, 1206b, 1302, 1302a, 1302b, 1308, 1308a, 1308b, 1400, 1400a, 1400b).

10. Procédé selon la revendication 9, dans lequel la détermination de si l'orientation du boîtier est l'orientation correspondant à la fonction de nettoyage comprend la détection d'un changement d'orientation angulaire du boîtier.

11. Procédé selon la revendication 9, comprenant en outre :
la production d'un premier niveau d'éclairage antimicrobien en réponse à la détermination du fait que le boîtier est dans une orientation non associée à la fonction de nettoyage ; et
la production d'un second niveau plus élevé d'éclairage antimicrobien en réponse à la détermination du fait que le boîtier est dans l'orientation correspondant à la fonction de nettoyage.

12. Procédé selon la revendication 9, comprenant en outre :
la détection du fait qu'un store configuré pour couvrir la zone cible et la source (1402, 1402a, 1402n) de l'éclairage antimicrobien est déployé ; et
le fait d'amener l'éclairage antimicrobien à un niveau de puissance plus élevé que lorsque l'éclairage antimicrobien est utilisé pour lire des indices lisibles par machine.

13. Procédé selon la revendication 12, comprenant en outre la communication d'un signal à tous les capteurs d'image et scanners (1102, 1102a, 1102b, 1108, 1108a, 1108b, 1110, 1206, 1206b, 1302, 1302a, 1302b, 1308, 1308a, 1308b, 1400, 1400a, 1400b) avec des sources d'éclairage antimicrobien (1402, 1402a, 1402n) pour émettre l'éclairage antimicrobien en réponse au déploiement du store à rouleau réfléchissant (1304).
